# EUROPÄISCHE PATENTANMELDUNG

(11) **EP 1 043 036 A2**
(43) Veröffentlichungstag der Anmeldung: **11.10.2000**
(21) Anmeldenummer: 00105777.7
(22) Anmeldetag: 18.03.2000
(51) Int. Cl.: A61M 1/16

(54) **Blut-Oxygenier-Vorrichtung**

(30) Priorität: 09.04.1999 DE 19916026
(71) Anmelder: Convergenza AG, 9490 Vaduz (LI)
(72) Erfinder: Stevens, Klaus, 52152 Simmerath (DE); Nellessen, Thomas, 78464 Konstanz (DE); Plechinger, Hans, Cranbrook, B.C. VIC 6J5 (CA)
(74) Vertreter: Vetter, Ewald Otto, Dipl.-Ing.

(57) **Zusammenfassung**

Blut-Oxygenier-Vorrichtung mit einem Druckregler (2) zur Regelung des Gasdruckes in Abhängigkeit vom Blutdruck. Das Gas dient über eine Gasaustausch-Membran zur Übertragung von Sauerstoff vom Gasstrom in den Blutstrom, und umgekehrt zur Übertragung von Kohlendioxid vom Blutstrom in den Gasstrom. Der Druckregler (2) hat einen einerseits vom Gasstrom und andererseits vom Blutstrom kontaktierten Ventilkörper (16,22), welcher in Abhängigkeit vom Differenzdruck zwischen den beiden Strömen mit einem den Gasstrom in die Außenatmosphäre entlüftenden Ventilsitz (28) zusammenwirkt.

## Beschreibung

Die Erfindung betrifft eine Blut-Oxygenier-Vorrichtung gemäß dem Oberbegriff von Anspruch 1.

Eine Blut-Oxygenier-Vorrichtung dieser Art ist aus der EP 0 245 784 B1 bekannt.

Weitere Blut-Oxygenier-Vorrichtungen sind aus den EP 0 373 847 A2, DE 43 20 198 C1, US 3 526 481 und WO 94/03266 (PCT/US93/07165) bekannt.

Durch die Erfindung soll die Aufgabe gelöst werden, die Blut-Oxygenier-Vorrichtung derart auszubilden, daß ihr Druckregler genauer und schneller reagierend arbeitet.

Diese Aufgabe wird gemäß der Erfindung durch die kennzeichnenden Merkmale von Anspruch 1 gelöst.

Die Erfindung wird verwirklicht durch eine Blut-Oxygenier-Vorrichtung, enthaltend einen Blutweg; einen Gasweg, der vom Blutweg getrennt ist; eine Gasaustausch-Membrananordnung zwischen einem Gaswegabschnitt und einem Blutwegabschnitt zum Gasaustausch zwischen dem Blut des Blutweges und dem Gas des Gasweges, wobei die Gasaustausch-Membrananordnung flüssigkeitsdicht, jedoch für den Gasaustausch gasdurchlässig ist; ein Druckregelventil, durch welches der im Gaswegabschnitt herrschende Gasdruck in Abhängigkeit von dem im Blutwegabschnitt herrschenden Blutdruck automatisch geregelt wird; dadurch gekennzeichnet, daß das Druckregelventil ein Gasrohr enthält, welches mit dem Gasweg strömungsmäßig verbunden ist und eine als Ventilsitz ausgebildete offene Stirnseite hat; daß der Ventilsitz außerhalb des Gasrohres mit der Außenatmosphäre strömungsmäßig verbunden ist; daß gegenüber dem Ventilsitz ein Ventilkörper angeordnet ist, welcher zum mehr oder weniger weiten Schließen oder Öffnen des Ventilsitzes in Gasrohr-Axialrichtung relativ zum Ventilsitz bewegbar ist; daß der Ventilkörper auf seiner von dem Ventilsitz abgewandten Seite in Richtung zum Ventilsitz hin mit einem dem jeweiligen Blutdruck im Blutweg entsprechenden Druck beaufschlagbar ist, so daß die Bewegung des Ventilkörpers relativ zum Ventilsitz durch den Differenzdruck zwischen dem Gasdruck im Gasrohr und dem Blutdruck im Blutweg bestimmt wird.

Das Druckregelventil der Erfindung arbeitet wesentlich exakter und hat kürzere Ansprechzeiten als die bekannten Regeleinrichtungen von Blut-Oxygenier-Vorrichtungen.

Weitere Merkmale der Erfindung sind in den Unteransprüchen enthalten.

Die Erfindung wird im folgenden mit Bezug auf die Zeichnungen anhand von bevorzugten Ausführungsformen als Beispiele beschrieben. In den Zeichnungen zeigen
- Fig. 1: einen Axialschnitt durch ein Druckregelventil einer Blut-Oxygenier-Vorrichtung nach der Erfindung,
- Fig. 2: einen Horizontalschnitt durch die Blut-Oxygenier-Vorrichtung in der Ebene II-II von Fig. 3,
- Fig. 3: eine Seitenansicht der Blut-Oxygenier-Vorrichtung,
- Fig. 4: eine Fig. 2 entsprechende Schnittdarstellung einer weiteren Ausführungsform einer Blut-Oxygenier-Vorrichtung nach der Erfindung,
- Fig. 5: eine Fig. 2 ähnliche Schnittdarstellung einer nochmals weiteren Ausführungsform einer Blut-Oxygenier-Vorrichtung nach der Erfindung.

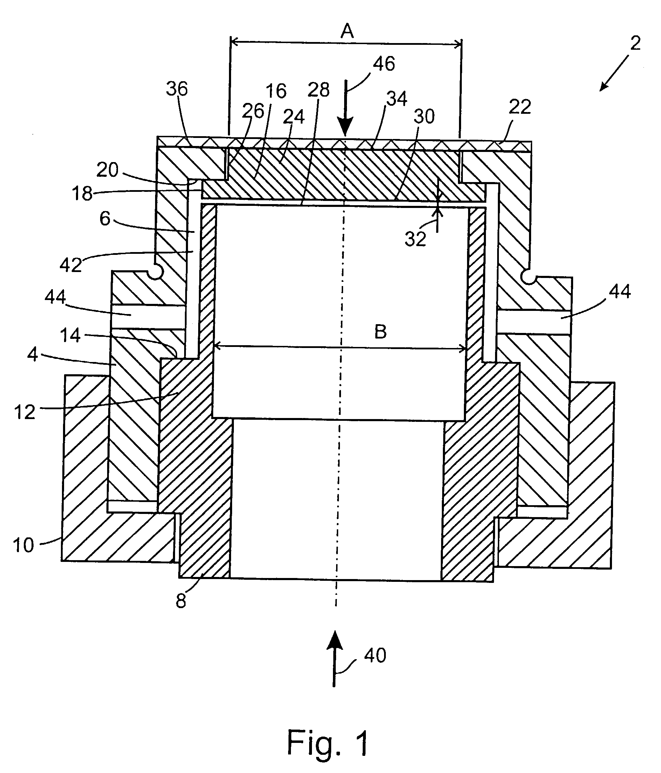

Fig. 1 zeigt einen Druckregler 2 der Blut-Oxygenier-Vorrichtung nach der Erfindung, wie sie in den folgenden Zeichnungen gezeigt ist.

Der Druckregler 2 kann in jeder beliebigen horizontalen, vertikalen oder schrägen Lage benutzt werden. Er besteht im wesentlichen aus einem rohrartigen Membranträger 4 mit einer Durchgangsöffnung 6; einem Gasrohr 8, welches von einer Seite her in die Durchgangsöffnung 6 eingesetzt ist und mit einer Überwurfmutter 10, welche auf den zylindrischen Membranträger 4 aufgeschraubt ist, an einem Ringbund 12 gegen einen Öffnungsabsatz 14 der Durchgangsöffnung 6 gespannt ist; einer Ventilplatte 16, welche vor dem Gasrohr 8 von der gleichen Seite wie dieses Gasrohr 8 in die Durchgangsöffnung 6 eingesetzt ist und mit einer radial überstehenden Ringrippe 18 auf einem zweiten Öffnungsabsatz 20 aufliegen kann, welcher einen kleineren Durchmesser als der erste Öffnungsabsatz 14 hat; und einer flexiblen, vorzugsweise auch elastisch dehnbaren Ventilmembran 22.

Die Ventilplatte 16 hat einen zylindrischen Kolbenabschnitt 24, welcher am anderen Ende der Durchgangsöffnung 6 in einem zylindrischen Bohrungsabschnitt 26 der Durchgangsöffnung 6 axial relativ zum Gasrohr 8 bewegbar geführt ist. Der zylindrische Bohrungsabschnitt 26 hat den kleinsten Durchmesser der Durchgangsöffnung 6 und befindet sich an dem anderen Ende der Durchgangsöffnung 6 als das Ende, in welches das Gasrohr 8 eingesetzt ist. Die Ringrippe 18 ragt um den gesamten Umfang radial über den Kolbenabschnitt 24 hinaus.

Die Stirnseite des in die Durchgangsöffnung 6 hineinragenden Endes des Gasrohres 8 ist als Ventilsitz 28 ausgebildet. Dieser Ventilsitz 28 ist von der inneren Stirnfläche 30 der Ventilplatte 16 mehr oder weniger weit oder vollständig verschließbar in Abhängigkeit von der axialen Position der Ventilplatte 16. Die axiale Dicke der Ringrippe 18 ist so bemessen, daß die Ringrippe 18, und damit die Ventilplatte 16, zwischen dem Ventilsitz 28 des Gasrohres 8 und dem zweiten Öffnungsabsatz 20 Axialbewegungen relativ zum ortsfesten Ventilsitz 28 machen kann. Die Strecke dieser Axialbewegungen liegt vorzugsweise im Bereich zwischen ungefähr 1/10 mm bis 4/10 mm, vorzugsweise bei etwa 2/10 mm.

Dadurch hat die innere Stirnfläche 30 von dem Ventilsitz 28 einen Abstand entsprechend der genannten Strecke, beispielsweise von 2/10 mm, wenn die Ringrippe 18 an dem zweiten Öffnungsabsatz 20 anliegt. Der Ventilöffnungsspalt 32 ist maximal so groß wie die genannte Strecke.

Bei größtem Öffnungsspalt 32 fluchtet die axial äußere Strinfläche 34 der Ventilplatte 16 mit der ihr benachbarten äußeren Stirnfläche 36 des rohrartigen Membranträgers 4. An dieser Stirnfläche 36 des Membranträgers 4 ist ein äußerer Umfangsbereich der folienartigen oder scheibenartigen Ventilmembran 22 befestigt, beispielsweise angeklebt oder anvulkanisiert. Sie erstreckt sich radial über das Ende der Durchgangsöffnung 6. Die Ventilmembran 22 liegt an der äußeren Stirnfläche 34 der Ventilplatte 16 an, ist jedoch vorzugsweise an ihr nicht befestigt.

Oxygenierungs-Druckgas 40 (Druckluft, reiner Sauerstoff, mit Sauerstoff angereichertes Gas oder andere Gasgemische) kann an dem vom Ventilsitz 28 abgewandten Ende in das Gasrohr 8, dann durch den Ventilspalt 32 über einen Ringraum 42 und dann durch Radialbohrungen 44, die im Membranträger 4 gebildet sind, in die Außenatmosphäre strömen. Der Ringraum 42 ist durch die einander benachbarten Umfangswände des Gasrohres 8 und des Membranträgers 4 zwischen den beiden Öffnungsabsätzen 14 und 20 gebildet. Die Radialbohrungen 44 führen von dem Ringraum 42 in die Außenumgebung. Durch den Druck von Blut 46 der Blut-Oxygenier-Vorrichtung auf der vom Ventilsitz 28 abgewandten Außenseite der Ventilmembran 22 wird die Ventilplatte mit ihrer inneren Stirnfläche 30 mehr oder weniger weit oder vollständig bis zum Ventilsitz 28 bewegt, entgegen dem Druck des Gases 40. Der Ventilspalt 32 ist vollständig geschlossen, wenn die Kraft auf der Seite des Blutes 46 größer ist als die Kraft auf der Seite des Gases 40. Wenn sich die Kraft des Gases 40 an die Kraft des Blutes 46 annähert, dann drückt das Gas den Ventilkörper 16 von dem Ventilsitz 28 weg und das Gas kann durch den Ventilspalt 32 in die Außenatmosphäre entweichen. Dadurch folgt der Gasdruck in einem durch die Ventilquerschnitte bestimmten Verhältnis dem Blutdruck. Die Ventilquerschnitte sind vorzugsweise derart gewählt, daß der Gasdruck maximal den Blutdruck erreichen, jedoch nicht darüber ansteigen kann.

Der Wert, um welchen der Gasdruck unterhalb des Blutdruckes gehalten wird, ist abhängig von dem Querschnittsverhältnis A:B. Hierbei ist A die Querschnittsfläche des zylindrischen Kolbenabschnittes 24 und B der Innenquerschnitt des Gasrohres 8 an dem Ventilsitz 28. Durch Verwendung eines anderen Querschnittes A:B kann der Druck des Gases höher als der Druck des Blutes eingestellt werden.

Vorzugsweise wird die Ventilplatte 16 weder in die eine noch in die andere axiale Richtung durch Federmittel vorbelastet. Durch die Vermeidung solcher Federmittel, wie dies bei der Ausführungsform von Fig. 1 der Fall ist, ergibt sich der Vorteil, daß der "Sicherheitsabstand" zwischen dem Blutdruck und dem Gasdruck prozentual um so größer wird, je höher der Blutdruck ist.

Der Druckregler 2 dient dazu, in der Blut-Oxygenier-Vorrichtung den Gasdruck des Gases 40 relativ zum Druck des Blutes 46 im Bereich einer Gasaustausch-Membran auf einem gewünschten, vorzugsweise einstellbaren Verhältnis zu halten. Der Druckregler 2 sollte möglichst nahe bei der Gasaustausch-Membran im Blutweg angeordnet werden, weil an anderen Stellen des Blutweges und/oder des Gasweges in der Blut-Oxygenier-Vorrichtung andere Druckwerte herrschen können, die bei dortiger Anordnung des Druckreglers 2 zu einem falschen Druck des Gases im Bereich der Gasaustausch-Membran führen könnten.

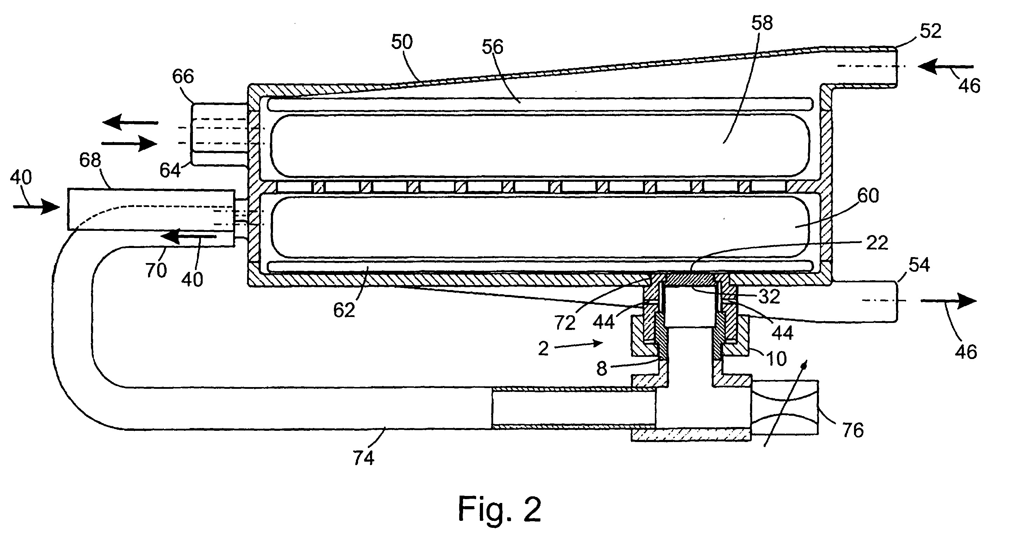

Die in Fig. 2 gezeigte Blut-Oxygenier-Vorrichtung enthält in einem Gehäuse 50 zwischen einem Bluteinlaß 52 und einem Blutauslaß 54 nacheinander einen Strömungsverteiler 56, einen Wärmetauscher 58, eine Gasaustausch-Membran 60 und einen Strömungssammler 62 für das Blut 46.

Der Wärmetauscher 58 hat einen Temperierwassereinlaß 64 und einen Temperierwasserauslaß 66.

Die Gasaustausch-Membran 60 kann jede beliebige Form haben und besteht vorzugsweise aus einem Bündel einer großen Vielzahl von Kapillarrohren, welche von dem Gas innen durchströmt werden und außen vom Strom des Blutes 46 umströmt werden. Durch die Gasaustausch-Membran 60 gelangt Sauerstoff von diesem Gasstrom in den Blutstrom, und umgekehrt Kohlendioxid vom Blutstrom in den Gasstrom. Die Blut-Oxygenier-Vorrichtung hat somit die Funktion einer Lunge.

Der Gasweg des Gases 40 hat am Gehäuse 50 einen, an eine Druckgasquelle anschließbaren Gaseinlaß 68 und einen Gasauslaß 70.

Bei der Ausführungsform nach Fig. 2 ist der Druckregler 2 an einer Wand des Gehäuses 50 derart befestigt, daß die Ventilmembran 22 in einer Wandöffnung 72 des Gehäuses 50 den Strom des Blutes 46 an einer Stelle seitlich begrenzt, welche zwischen der Gasaustausch-Membran 60 und dem Blutauslaß 54 liegt.

Der Gasauslaß 70 ist durch eine Leitung 74 an das Gasrohr 8 des Druckreglers 2 angeschlossen.

Das Gasrohr 8 oder die an es angeschlossene Gasauslaßleitung 74 kann über eine einstellbare Drossel 76 mit der Außenatmosphäre verbindbar sein. Wenn die Drossel 76 vollständig geöffnet ist, hat der Druckregler 2 keine Regelwirkung mehr. Wenn die Drossel 76 vollständig geschlossen ist, ergibt sich im Druckregler 2 der größtmögliche Gasdruck. Somit kann durch die Drossel 76 der "Arbeitsdruck" des Druckreglers 2 eingestellt werden, entweder manuell oder automatisch in Abhängigkeit von einer elektronischen Steuereinrichtung, beispielsweise einer Herz-Lungen-Maschine.

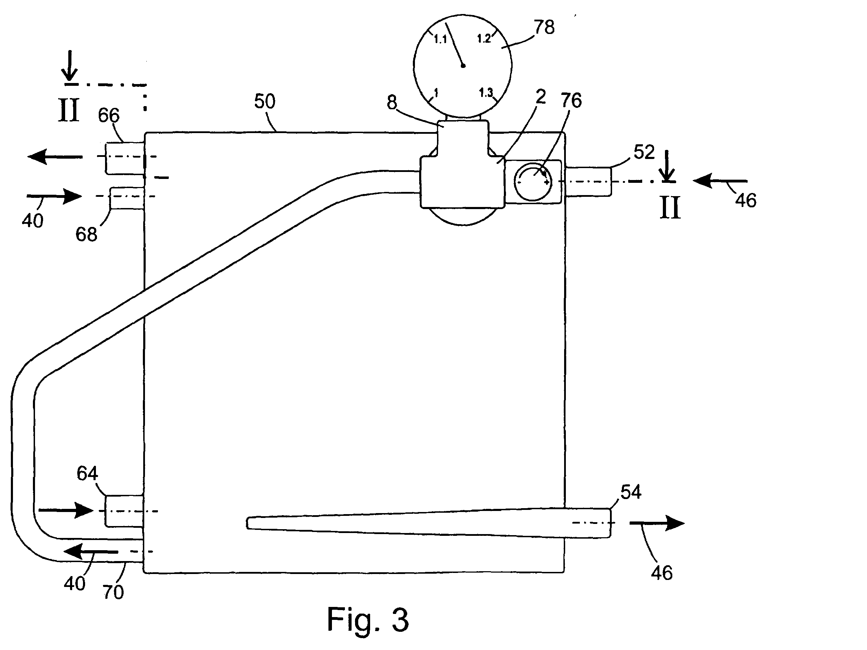

Gemäß Fig. 3 kann an das Gasrohr 8 des Druckreglers 2 ein Druckanzeigegerät 78 (Manometer) angeschlossen sein.

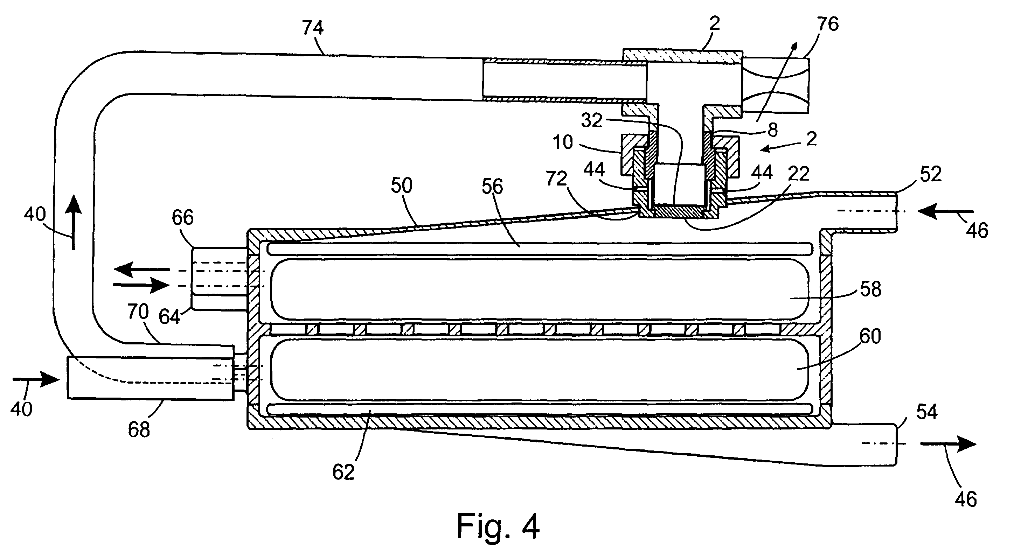

Die Ausführungsform nach Fig. 4 unterscheidet sich von der Ausführungsform nach den Fig. 1 bis 3 nur dadurch, daß der Druckregler 2 so angeordnet ist, daß seine Ventilmembran 22 in dem Gehäuse 50 nahe dem Bluteinlaß 52 dem Blutstrom 46 ausgesetzt ist.

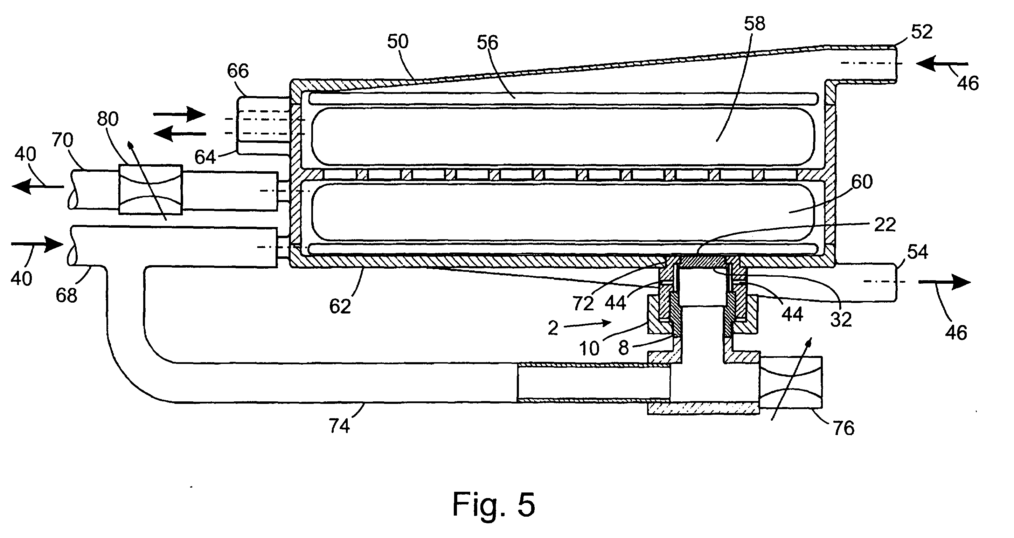

Die in Fig. 5 gezeigte Ausführungsform unterscheidet sich von den Fig. 1 bis 3 lediglich dadurch, daß nicht der Gasauslaß 70, sondern der Gaseinlaß 68 der Gasaustausch-Membran 60 an das Gasrohr 8 des Druckreglers 2 angeschlossen ist. Bei der Ausführungsform von Fig. 5 muß die Drossel 76 am Eingang des Druckreglers 2 vollständig geschlossen sein, und der Gasauslaß 70 muß durch eine, vorzugsweise einstellbare, Drossel 80 so gedrosselt werden, daß im Oxygenator-Gehäuse 50 der gewünschte Gasüberdruck entsteht.

Soweit nicht im Detail beschrieben, sind bei den Ausführungsformen nach den Fig. 4 und 5 die gleichen Elemente und die gleichen Funktionen vorgesehen, wie dies mit Bezug auf die Fig. 1 bis 3 beschrieben wurde.

## Patentansprüche

1. Blut-Oxygenier-Vorrichtung, enthaltend einen Blutweg; einen Gasweg, der vom Blutweg getrennt ist; eine Gasaustausch-Membrananordnung (60) zwischen einem Gaswegabschnitt und einem Blutwegabschnitt zum Gasaustausch zwischen dem Blut (46) des Blutweges und dem Gas (40) des Gasweges, wobei die Gasaustausch-Membrananordnung (60) flüssigkeitsdicht, jedoch für den Gasaustasch gasdurchlässig ist; ein Druckregelventil (2), durch welches der im Gaswegabschnitt herrschende Gasdruck in Abhängigkeit von dem im Blutwegabschnitt herrschenden Blutdruck automatisch geregelt wird und welches ein Gasrohr (8) enthält, dessen Gasrohrinnenraum mit dem Gasweg strömungsmäßig verbunden ist; **dadurch gekennzeichnet**, daß das Gasrohr (8) eine als Ventilsitz (28) ausgebildete offene Stirnseite hat, welche neben dem Ventilsitz (28) außerhalb des Gasrohres (8) mit der Außenatmosphäre strömungsmäßig verbunden ist; daß gegenüber dem Ventilsitz (28) ein Ventilkörper (16,22) angeordnet ist, welcher zum mehr oder weniger weiten Schließen oder Öffnen des Ventilsitzes (28) in Gasrohr-Axialrichtung relativ zum Ventilsitz bewegbar ist, um den Gasrohrinnenraum in die Außenatmosphäre zu entlüften, die sich außerhalb des Gasrohres (8) befindet und sich dort an den Ventilsitz (28) anschließt, daß der Ventilkörper (16,22) auf seiner von dem Ventilsitz (28) abgewandten Seite einen Kolbenabschnitt (24) aufweist, auf welchen der Druck des Blutweges in Richtung zum Ventilsitz hin wirkt, so daß die Position des Ventilkörpers relativ zum Ventilsitz durch den Differenzdruck zwischen dem Gasdruck im Gasrohr (8) und dem Blutdruck im Blutweg und durch das Verhältnis des vom Blutdruck des Blutweges beaufschlagten Kolbenquerschnitts (A) und des Innenquerschnitts (B) des Gasrohres (8) am Ventilsitz (28) bestimmt wird.

2. Blut-Oxygenier-Vorrichtung nach Anspruch 1, **dadurch gekennzeichnet**, daß der Kolbenabschnitt (24) auf seiner vom Ventilsitz (28) abgewandten Seite von einer flexiblen Ventil-Membran (22) überdeckt ist, die ihn vom Blut des Blutweges trennt und radial außerhalb des Kolbenabschnittes (24) an einem Membranträger (4) befestigt ist, der relativ zum Ventilsitz (28) ortsfest angeordnet ist.

3. Blut-Oxygenier-Vorrichtung nach Anspruch 2, **dadurch gekennzeichnet**, daß der Kolbenabschnitt (24) in dem Membranträger (4) in Axialrichtung des Gasrohres (8) bewegbar geführt angeordnet ist.

4. Blut-Oxygenier-Vorrichtung nach Anspruch 2 oder 3, **dadurch gekennzeichnet**, daß die Ventil-Membran (22) und die Ventilplatte (16) einander kontaktierbar angeordnet sind, jedoch nicht aneinander befestigt sind.

5. Blut-Oxygenier-Vorrichtung nach einem der Ansprüche 2 bis 4, **dadurch gekennzeichnet**, daß der Membranträger (4) ein rohrartiges Gehäuse ist, welches eine Durchgangsöffnung (6) hat, an deren einem Ende die Ventil-Memebran (22) befestigt ist und durch deren anderes Ende das Gasrohr (8) in die Durchgangsöffnung hineinragt, wobei sich die Rohr-Strinseite, welche den Ventilsitz (28) bildet, in der Durchgangsöffnung (6) befindet.

6. Blut-Oxygenier-Vorrichtung nach Anspruch 5, **dadurch gekennzeichnet**, daß das Gasrohr (8) an dem Membranträger (4) befestigt ist.

7. Blut-Oxygenier-Vorrichtung nach einem der vorhergehenden Ansprüche, **dadurch gekennzeichnet**, daß eine Strömungsdrossel (76) vorgesehen ist, durch welche das Gasrohr (8) in die Atmosphäre entlüftbar ist.

8. Blut-Oxygenier-Vorrichtung nach Anspruch 7, **dadurch gekennzeichnet**, daß der Strömungsquerschnitt der Strömungsdrossel (76) variabel einstellbar ist.

9. Blut-Oxygenier-Vorrichtung nach einem der vorhergehenden Ansprüche, **dadurch gekennzeichnet**, daß der Ventilkörper (16,22) auf seiner vom Ventilsitz abgewandten Seite eine Wand des Blutweges bildet, so daß er von dessen Blut kontaktierbar ist.

10. Blut-Oxygenier-Vorrichtung nach einem der vorhergehenden Ansprüche, **dadurch gekennzeichnet**, daß der Ventilkörper (16,22) an einer Wandöffnung eines Gehäuses (50) angeordnet ist, in welcher die Gasaustausch-Membrananordnung (60) untergebracht ist, wobei das Gehäuse (50) und die Gasaustausch-Membrananordnung zwischen sich den Blutwegabschnitt für den Gasaustausch begrenzen.

11. Blut-Oxygenier-Vorrichtung nach einem der vorhergehenden Ansprüche, **dadurch gekennzeichnet**, daß der Ventilkörper (16,22) auf der stromabwärtigen Blutwegseite der Gasaustausch-Membrananordnung (60) angeordnet ist.

12. Blut-Oxygenier-Vorrichtung nach einem der Ansprüche 1 bis 10, **dadurch gekennzeichnet**, daß der Ventilkörper (16,22) auf der stromaufwärtigen Blutwegseite der Gasaustausch-Membrananordnung (60) angeordnet ist.

13. Blut-Oxygenier-Vorrichtung nach einem der vorhergehenden Ansprüche, **dadurch gekennzeichnet**, daß das Gasrohr (8) mit der stromabwärtigen Gaswegseite der Gasaustausch-Membrananordnung (60) strömungsmäß9g verbunden ist.

14. Blut-Oxygenier-Vorrichtung nach einem der Ansprüche 1 bis 12, **dadurch gekennzeichnet**, daß das Gasrohr (8) mit der stromaufwärtigen Gaswegseite der Gasaustausch-Membrananordnung (60) strömungsmäßig verbunden ist.
